# EUROPEAN PATENT APPLICATION

(11) **EP 4 354 459 A1**
(43) Date of publication of application: **17.04.2024**
(21) Application number: 23199660.4
(22) Date of filing: 26.09.2023
(51) Int. Cl.: G16H 80/00, G16H 40/67, G16H 50/20, G16H 50/30, G16H 10/20, G16H 10/60, G16H 20/30, G16H 20/10, A61B 5/11, A61B 5/00

(54) **MONITORING THE DISEASE PROGRESSION OF A PARKINSON'S PATIENT**

(30) Priority: 13.10.2022 US 202263415827 P
(71) Applicant: Bayer AG, 51373 Leverkusen (DE)
(72) Inventor: Podhaisky, Hans-Peter, 13156 Berlin (DE); Schröder, Jens, 13467 Berlin (DE); Gøtzsche, Casper, 3060 Espergærde (DK); Kremliovsky, Michael, Poway, 92064 (US); Pandya, Purva, Spring Grove, 17362 (US)
(74) Representative: BIP Patents

(57) **Abstract**

The disclosure relates to systems, methods, and computer programs for monitoring the disease progression of a Parkinson's patient.

## Description

### FIELD

Systems, methods, and computer programs disclosed herein relate to monitoring the disease progression of a Parkinson's patient.

### BACKGROUND

Parkinson's disease ("PD") is a progressive neurological condition resulting from the loss of dopaminergic neurons in a specialized area of the brain called the substantia nigra. PD can present at any age, however less than 10% of cases are reported before the age of 40. The majority of PD patients are elderly people, and this population cohort is expanding rapidly. Thus, PD is becoming an increasing public health and socioeconomic challenge. The primary issues in PD are due to reduced capacity for self-care, performance of activities of daily living and reduced quality of life.

The symptoms of PD can be broadly classified into motor symptoms and non-motor symptoms. Motor symptoms (MS) include rigidity, tremor, bradykinesia (slow movement) and postural alterations. These symptoms are cardinal features of PD and contribute to progressive gait disturbances, which are strongly linked to a reduced quality of life. These gait disturbances present in terms of a reduction in stride length and walking speed, increased stride variability and episodes called freezing of gait.

Gait disturbances often lead to falls and hospitalization.

Diagnosis and monitoring of patients with Parkinson's disease is crucial for assessing the severity of the disease progression in order to respond with appropriate preventive measures. In the event of Parkinson's disease onset, timely intervention can be lifesaving. Comprehensive monitoring to assess PD remains a significant unmet clinical need.

Parkinson's disease cannot yet be cured, but medications can help control the symptoms, often dramatically.

Pharmacologic treatments for Parkinson disease motor symptoms are primarily dopamine based. Levodopa preparations, dopamine agonists, and monoamine oxidase-B (MAO-B) inhibitors are useful initial therapies. For young individuals with prominent tremor, anticholinergic agents are useful, but caution is required because of the potential for adverse events, particularly relating to cognition.

Over time, individuals with Parkinson disease commonly require more frequent levodopa doses in addition to higher doses. This phenomenon is not due to medication tolerance or loss of efficacy of levodopa. As Parkinson disease progresses, individuals lose their long-duration response to dopaminergic medication, and their short-duration response decreases due to disease-related pathophysiologic changes in the brain. The brain also loses the ability to store extra dopamine (whether produced internally or provided through medication) for later use.

Finding the optimal dosage of medications to compensate for dopamine deficiency is difficult. On the one hand, the effect is highly dependent on the individual. Furthermore, a temporal profile of drug effect (accumulation phase, reduction phase) must be considered. The need and effect of active ingredients are also context-dependent, for example, on the patient's activity or the like. Ultimately, the different modes of action of different drugs influence each other, so that on the one hand the effect may be insufficient and on the other hand side effects may occur. Ultimately, the parameters relevant to the disease state, e.g., the level of dopamine in the brain in Parkinson's disease, cannot be measured directly.

The treating physician must therefore proceed experimentally. He/she is forced to act according to the "trial and error" principle. The patient is prescribed a pre-determined initial dose after which symptoms are awaited, i.e., the effect of this medication is only observed. An adjustment of the dose depending on this is then made iteratively.

### SUMMARY

The problems described above, and other problems are addressed by the present disclosure.

In a first aspect, the present disclosure provides a computer-implemented method, the method comprising:
- receiving specifications for a Parkinson's patient, wherein the specifications were defined by a physician based on an individual therapy for the patient, wherein the specifications contain one or more threshold values,
- at repeating points in time:
   ∘ outputting a message to the patient, the message comprising a request to perform a mobility test,
   ∘ determining and/or receiving one or more mobility parameters as a result of the performance of the mobility test,
   ∘ comparing the one or more mobility parameters with the one or more threshold values, thereby determining one or more deviations between the one or more mobility parameters and the one or more threshold values,
- making available the mobility parameters and/or the deviations to the physician by outputting and/or storing the mobility parameters and/or the deviations, and/or by transmitting the mobility parameters and/or the deviations to a separate computer system.

In another aspect, the present disclosure provides a computer system, the computer system comprising: a processor, an input unit, an output unit, and a memory storing an application program configured to perform, when executed by the processor, an operation, the operation comprising:
- receiving via the input unit specifications for a Parkinson's patient, wherein the specifications were defined by a physician based on an individual therapy for the patient, wherein the specifications contain one or more threshold values,
- at repeating points in time:
   ∘ causing the output unit to output a message to the patient, the message comprising a request to perform a mobility test,
   ∘ determining and/or receiving via the input unit one or more mobility parameters as a result of the performance of the mobility test,
   ∘ comparing the one or more mobility parameters with the one or more threshold values, thereby determining one or more deviations between the one or more mobility parameters and the one or more threshold values,
- making available the mobility parameters and/or the deviations to the physician by outputting and/or storing the mobility parameters and/or the deviations, and/or by transmitting the mobility parameters and/or the deviations to a separate computer system.

In another aspect, the present disclosure provides a non-transitory computer readable medium having stored thereon software instructions that, when executed by a processor of a computer system, cause the computer system to execute the following steps:
- receiving specifications for a Parkinson's patient, wherein the specifications were defined by a physician based on an individual therapy for the patient, wherein the specifications contain one or more threshold values,
- at repeating points in time:
   ∘ outputting a message to the patient, the message comprising a request to perform a mobility test,
   ∘ determining and/or receiving one or more mobility parameters as a result of the performance of the mobility test,
   ∘ comparing the one or more mobility parameters with the one or more threshold values, thereby determining one or more deviations between the one or more mobility parameters and the one or more threshold values,
- making available the mobility parameters and/or the deviations to the physician by outputting and/or storing the mobility parameters and/or the deviations, and/or by transmitting the mobility parameters and/or the deviations to a separate computer system.

Further aspects of the present invention are disclosed in the dependent claims, the specification, and the drawings.

### DETAILED DESCRIPTION

The invention will be more particularly elucidated below without distinguishing between the aspects of the invention (method, computer system, computer-readable storage medium). On the contrary, the following elucidations are intended to apply analogously to all the aspects of the invention, irrespective of in which context (method, computer system, computer-readable storage medium) they occur.

If steps are stated in an order in the present description or in the claims, this does not necessarily mean that the invention is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides a digital tool for a patient suffering from Parkinson's disease and for a physician to track the progression of the disease.

The digital tool can be used in decentralized clinical trials for the development of therapies against Parkinson's disease as well as for therapy monitoring of Parkinson's patients.

It has the advantage of being able to monitor patients in their home environment over a long period of several weeks, months or even years.

The digital tool provides the physician with information about the patient's health status, the progression of Parkinson's disease, and the effectiveness of therapy in the form of easily determined digital biomarkers.

Digital biomarkers are defined as objective, quantifiable physiological and behavioral data that are collected and measured by means of digital devices such as portables, wearables, implantables, or digestibles.

A digital tool is understood to be a computer program that can be executed on one or more computer system(s).

The digital tool may also include multiple computer programs, such as a first computer program used by a physician on a first computer system (the physician's computer system) and a second computer program used by the patient on a second computer system (the patient's computer system).

There may be one or more interfaces between the multiple computer programs (e.g., between the first computer program and the second computer program) for passing data generated, computed, received, and/or determined by one computer program to the other computer program.

For simplicity, the invention is explained below with reference to a single computer program used by the physician and the patient, without limiting the invention to this embodiment.

In a first step, the computer program is configured by the physician for the patient.

The configuration includes entering one or more parameters into the computer system, wherein the one or more parameters are determined by the physician based on the patient's disease state, past medical history, chosen therapy, and/or the patient's needs.

The parameters to be entered by the physician include one or more threshold values for a mobility test that the patient is to perform repeatedly at defined intervals.

Preferably, the mobility test is one that the patient can perform without assistance in his or her home environment or while traveling.

For example, the mobility test can be a timed "up and go test".

In the timed "up and go" test, the patient is asked to walk a certain distance from a resting position and return to a resting position. The resting position can be, for example, a sitting position on a chair with or without armrests. The defined distance may be, for example, a distance of three meters in one direction and back. For example, the patient may be asked to stand up from a sitting position on a chair with armrests, walk a distance of three meters in one direction away from the chair, turn around, walk back to the chair, and sit down again.

For example, the one or more thresholds may include a time period, such as a maximum time the patient should take to complete the mobility test (e.g., the timed up and go test).

The one or more thresholds may include, for example, a distance, such as the minimum distance the patient should walk during a defined period of time.

The one or more thresholds may include, for example, a minimum number of exercises that the patient should perform as part of the mobility test (for example, the number of exercises in a given period of time).

The parameters to be entered by the physician may include the type of mobility test, the time at which the mobility test is to be performed (e.g., by specifying one or more points in time or time spans), and/or the frequency of the mobility test (i.e., how often (at which intervals) the patient needs to perform the mobility test). It is conceivable that several (different) mobility tests are to be performed.

Once the computer program is configured, it can be used by the patient.

The computer program can remind the patient to perform the mobility tests and/or monitor the performance of the mobility tests. It is intended that the patient performs the mobility test(s) repeatedly at defined times or within defined time periods.

In one embodiment, the computer program comprises an alarm function that reminds the patient to perform the mobility test.

For example, the computer program can cause the patient's computer system to output a message through a speaker (e.g., a tone or sequence of tones or a voice message), output a message on a screen, and/or use a vibration alert to remind the patient to perform the mobility test.

In the message, the computer program may ask the patient to prepare to perform the mobility test. If the mobility test is a timed up and go test, the message may prompt the patient to sit in a chair. In the message, the computer program may use text, speech, images, animations, and/or video sequences to explain what the patient is to do as part of the mobility test.

The message can include a request to the patient to indicate whether he or she is ready to perform the mobility test. The patient can indicate his or her readiness, e.g., by a voice command or by pressing a virtual button on the screen.

It is possible that the patient initiates the start of the mobility test by entering a command into the computer system (e.g., by pressing a virtual button and/or by a voice command); however, it is also possible that the computer system indicates the start. For example, a countdown may be displayed on a screen and/or output through a speaker. After the countdown has elapsed, the patient should start the mobility test.

It is also conceivable that the start of the mobility test is detected by the computer system, for example by means of one or more sensors.

Such a sensor may be, for example, a motion sensor (e.g., an activity tracker). Many cell phones (smartphones), tablet computers, and smart watches include motion, gyroscopic and/or accelerometer sensors that can detect patient activity. The computer system of the present disclosure may be or comprise such a cell phone (smartphones), tablet computer, or smart watch.

However, it is also possible for the patient to wear one or more motion, gyroscopic and/or acceleration sensor(s) as wearable sensor(s) on his/her body. The wearable sensor(s) can transmit data about the patient's movement to the patient's computer system via a wireless connection, for example.

If a rest period is at the beginning of the mobility test, the computer system may be configured to detect patient activity indicating the end of the rest period and the beginning of the mobility test.

The computer system may be configured to start a stopwatch (timer) at the beginning (start) of the mobility test.

However, it is also possible that the patient starts the stopwatch at the beginning of the mobility test, e. g., by pressing a virtual button on a screen of a computer system and/or by a voice command It is also possible for the stopwatch to start automatically, e.g., after the countdown has expired and/or after the patient has been prompted to start the mobility test.

The mobility test can be completed when the patient returns to a resting position.

The end of the mobility test can be indicated by the patient (e.g., by pressing a virtual switch and/or by a voice command) and/or detected by one or more sensors (e.g., motion sensor and/or gyroscopic sensor and/or accelerometer).

At the end of the mobility test, the stopwatch can be stopped and the time for performing the mobility test can be saved (e.g., in a data memory of the computer system).

The beginning and/or end of the mobility test can also be monitored with a video camera, which may be part of the computer system or connected to it via a wired or wireless connection.

The computer system may also be configured to monitor the patient's performance of the mobility test.

Monitoring of the performance of the mobility test can be done to check if the patient is improving or deteriorating over time.

Monitoring of the performance of the mobility test can be performed to check whether a therapeutic measure against Parkinson's disease is successful in the patient (and the patient's performance improves or at least does not deteriorate when repeatedly performing the mobility test), or whether a therapeutic measure against Parkinson's disease is not successful in the patient (and the patient's performance deteriorates when repeatedly performing the mobility test).

The performance of the mobility test may be monitored to determine if a dosage of a medication is appropriate and/or if the amount of the medication needs to be increased or decreased and/or if the frequency of the patient's use of the medication needs to be increased or decreased.

In particular, if the patient performs the mobility test repeatedly over an extended period of weeks, months, and even years, the physician can monitor the progression of the disease and intervene as needed.

Monitoring the mobility test involves determining one or more mobility parameters.

For example, a mobility parameter may be the duration of the mobility test, i.e., the time it takes the patient to complete the mobility test.

A mobility parameter can be the distance a patient walks in one direction in a pre-defined time.

A mobility parameter can be the number of exercises a patient performs during a pre-defined time period.

Monitoring can be done with the help of a stopwatch and/or with the help of one or more sensors, such as motion sensor, gyroscopic sensor, accelerometer, camera, GPS receiver and/or distance sensor.

The stopwatch and/or the one or more sensors may be components of the computer system and/or connected to it via a wired connection or wirelessly.

The determined mobility parameter(s) can be displayed on a screen (e.g., for the patient and/or the physician), output via a loudspeaker, stored in a data memory and/or transmitted via a network to a separate computer system (e.g., the computer system used by the physician).

The determined mobility parameters can be compared to the one or more thresholds that the physician has set for the patient.

Comparing one or more mobility parameters to one or more thresholds may include determining a deviation of a mobility parameter from a threshold. For example, if the physician has determined for the patient that the patient should complete the mobility test in at least 20 seconds (threshold value = 20 seconds), and the patient takes 22 seconds, then the duration (actual value) is two seconds above the threshold value (the deviation is two seconds).

The determined deviation(s) can be displayed on a screen (e.g., for the patient and/or the physician), output via a loudspeaker, stored in a data memory and/or transmitted via a network to a separate computer system (e.g., the computer system used by the physician).

In the event that a mobility parameter deviates in a defined manner from a threshold set by the physician, a message may be sent to the patient requesting that he or she contact the physician (e.g., immediately or within a time period set by the physician or at the earliest opportunity).

In the event that a mobility value deviates in a defined manner from a threshold set by the physician, the computer system may be configured to transmit a message to the physician informing the physician of the deviation.

"In a defined matter" may mean that the mobility parameter is above the threshold value (e.g., if the threshold is a maximum time that the mobility test should last) or that the mobility parameter is below the threshold value (e. g., if the threshold is a minimum distance the patient should move or a minimum number of exercises the patient should perform in atime defined by the physician). "In a defined matter" may mean that the mobility parameter is above or below the threshold repeatedly (for example, for the second or third or fourth time in a row or within a period of time defined by the physician).

It is possible that such a message is sent automatically. It is also possible that such a message is sent only after an action of the patient. For example, the computer system may be configured to issue a message to the patient informing the patient that a mobility parameter deviates in a defined manner from a threshold set by the physician. The patient may be prompted to initiate the transmission of a message to the physician about the deviation by pressing a virtual switch and/or by a voice command. The patient may be prompted to consent to transmission of the one or more determined mobility parameters and/or deviations to the physician.

It is also possible that one or more mobility parameters determined during the performance of one or more mobility tests by the patient and/or one or more deviations can be output to the physician during a visit of the patient to the physician. For this purpose, the physician can, for example, connect the patient's computer system to his/her own computer system, transfer the mobility parameter(s) and/or deviation(s) stored on the patient's computer system to his/her computer system and have them output on his/her computer system.

Based on the transferred data, the physician can then check whether the patient's health has improved, remained the same or worsened. The physician can check whether the therapy he/she has chosen is working for the patient. The physician can check whether the dosage of a medication (i.e., the amount and/or frequency of taking it) is correct (i.e., leads to the desired success) or needs to be adjusted.

A (repeated) defined deviation of one or more mobility parameters from one or more threshold values may lead to a change in a therapeutic intervention.

If one or more mobility parameters for the repeated time deviate in a defined manner from one or more threshold values, this may mean that the dose of a medication for Parkinson's disease is too low and/or it needs to be taken more frequently. It is possible that the computer system is configured to issue a message to the patient stating that the patient should take a modified amount of a medication and/or take the medication more frequently in the future and/or take another and/or further medication.

The computer system may be configured to issue a message to the patient after (each) completion of a mobility test, providing feedback to the patient about the patient's performance on the mobility test.

For example, if the patient has deteriorated compared to the last time the mobility test was performed and/or compared to the average of a series of times the mobility test was previously performed (e.g., by taking longer to perform the mobility test), the computer system may be configured to output a message to the patient indicating that the performance was not as good as the last time the mobility test was performed (or the average of a series of times the mobility test was previously performed) and encouraging the patient that it will certainly be better next time.

However, it is also conceivable that the physician will determine whether a deterioration in performance is indicated as such to the patient. Especially for people suffering from Parkinson's disease, it can be important not to discourage them with negative messages about deterioration in their health.

Conversely, the computer system may be configured to indicate as such to the patient any improvement in the patient's performance in performing the mobility test. The computer system may be configured to issue a message to the patient upon completion of a mobility test, praising the patient if his or her condition has improved or at least not worsened.

Such positive feedback can be very important for Parkinson's patients and give them courage.

In an embodiment, monitoring of the performance of the mobility test is performed to verify that the patient is performing the mobility test correctly.

For example, if the mobility test involves walking a defined distance in one direction, the computer system may be configured to use the one or more sensors to monitor the mobility test and verify that the patient is walking the defined distance. "Using the one or more sensors to monitor" may mean that the one or more sensors are configured to transmit sensor data to the computer system and the computer system is configured to receive the sensor data.

If the verification shows that the patient performed the mobility test as intended, the computer system may be configured to output a message to the patient confirming that the mobility test was performed correctly.

If the verification shows that the patient did not perform the mobility test as intended, the computer system may be configured to output a message to the patient indicating that the mobility test was not performed correctly. The message may include information about what caused the computer system to indicate that the mobility test was not performed correctly (for example, the distance walked may be too great or too small).

If the mobility test was not performed correctly, the patient can be prompted to repeat the mobility test immediately or after a specified time. If the patient is asked to repeat the mobility test after a specified time, an alarm function can remind the patient to repeat the mobility test at the appropriate time.

It is possible that in addition to the mobility parameters determined during the performance of the mobility test, further parameters are recorded by and/or with the help of the patient's computer system Such further parameters may include one or more biomarkers, or one or more biomarkers may be determined (e.g., calculated) by the computer system based on such further parameters.

Therefore, such further parameters can be used, in addition to the one or more mobility parameters, to determine the patient's health status, to check whether the health status has improved, worsened or remained the same, to check the effect of a therapy, and/or to check whether the prescribed dosage of a drug for Parkinson's symptoms is correct or needs to be changed.

Such further parameters are also preferably recorded and/or determined in the patient's home environment.

For example, it is possible that the patient's activity in daily life is detected/recorded by means of one or more motion, gyroscopic and/or acceleration sensors.

The one or more sensors may be part of and/or connected to the patient's computer system via a wired and/or wireless connection.

The sensor data (activity data) can be recorded in real time with an adjustable rate, as an example, of 10 Hz to 50 Hz. The sensor data (activity data) can be analyzed locally on the patient's computer system or remotely on a separate computer system (e.g., the physician's computer system and/or through cloud services).

The sensor data (activity data) can be used to create an activity profile for the patient. It is possible to assign the patient to a profile based on activity data. Activity profiles are described, for example, in: P. von Rosen et al.: Physical activity profiles in Parkinson's disease, BMC Neurology 2021, 21, 71; K.P. Dowd et al.: A systematic literature review of reviews on techniques for physical activity measurement in adults: a DEDIPACstudy, Int J Behav Nutr Phys Act. 2018, 15(1), 15; R. Mesquita et al.: Physical activity patterns and clusters in 1001 patients with COPD, Chron Respir Dis. 2017, 14(3), 256-69; H.E.M. Braakhuis et al.: Three distinct physical behavior types in fatigued patients with multiple sclerosis, J Neuroeng Rehabil. 2019, 16(1), 105.

A change in activity profile over time can mean an improvement and/or deterioration in health status.

Recording activity data can also help motivate patients to exercise more. Exercise has a positive influence on the course of the disease. Messages can be issued to the patient when he/she has completed a minimum number of steps or other activity. The patient can be reminded to exercise more by a message.

Activity data and/or an activity profile determined for the patient can be output to the physician.

A further parameter may be the sleep profile of the patient. The sleep profile of a Parkinson's patient can provide information about the status and progression of the disease. The sleep profile can be recorded with activity trackers, for example (see, e.g., W. S chrempfet al.: Sleep disorders in Parkinson's disease, J. Parkinsons Dis. 2014, 4(2), 211-221).

The sleep profile can be output to the physician.

Other parameters that can be collected through the patient's computer system can be collected with a quality-of-life (QoL) questionnaire.

Such questionnaires can capture the patient's subjective perception of health and support therapy planning.

A disease specific QoL instrument for use with patients with Parkinson's disease, the PDQ-39, has been shown to have good reliability, validity, responsiveness, and reproducibility, and is used in many trials to assess effectiveness of treatment (see, e.g., R. Fitzpatrick et al.: Desirable properties for instruments assessing quality of life: evidence from the PDQ-39, J Neurol Neurosurg Psychiatry 1997, 62, 104).

The questions can be presented to the patient at set times at recurring intervals (e.g., every evening) and/or at specific events (e.g., after detecting a period of rest lasting longer than a predefined period of time (e.g., 1 hour)) and/or after an activity lasting a predefined minimum time (e.g., 1 hour).

The patient's answers to the QoL questions can be issued to the physician.

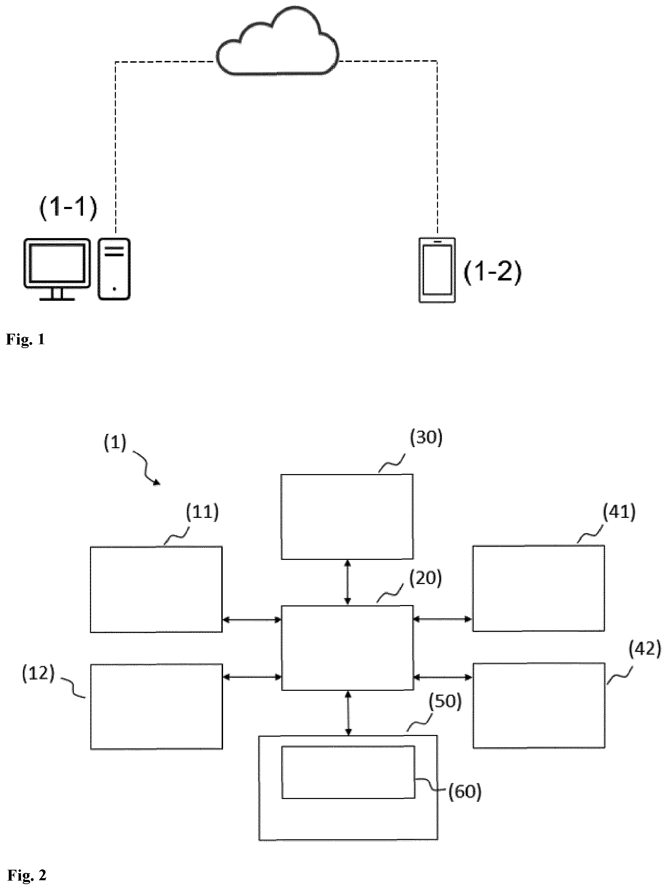

Fig. 1 shows an exemplary and schematic embodiment of the system of the present disclosure. The system includes two computer systems, a first computer system (1-1) used by a physician and a second computer system (1-2) used by a patient.

The first computer system (1-1) and the second computer system (1-2) are connected via a network (represented by the dashed lines and the cloud).

In the first computer system (1-1), the doctor can enter specifications for the Parkinson's patient. For example, he/she can specify which mobility test the patient should perform, when the patient should perform it, and/or at what time interval(s) the patient should perform it.

The physician can enter one or more threshold values for the mobility test(s).

The physician can specify which medication the patient should take, when the patient should take the medication, the amount the patient should take, and how often the patient should take the medication.

The physician can specify which questionnaire and/or questions the patient should answer on a regular basis about the quality of the patient's life, when the patient should answer the questions, and/or how often the patient should answer the questions.

The specifications can then be transferred over the network from the first computer system (1-1) to the second computer system (1-2).

In an alternative embodiment, there is only one computer system in which the physician sets the specifications and which the physician then gives to the patient for use.

The second computer system (1-2) can remind the patient to take the medication. The second computer system (1-2) may send a message to the patient at the specified time informing the patient which medication and/or which amount of the medication the patient should take. Preferably, the second computer system (1-2) prompts the patient to confirm that the medication is to be taken, for example, by pressing a virtual button and/or by a voice command. The second computer system (1-2) may also be configured to track the patient taking the medication using a camera (see, e.g., G. A. Bilodeau et al.: Monitoring of Medication Intake Using a Camera System, J Med Syst 2011, 35, 377-389).

By confirming or monitoring the patient's medication intake, the physician can verify at a later time whether and/or when the patient took the medication and whether the patient took the medication at the specified times. If there are discrepancies between the specified and actual times the medication was taken, this is an indication to the physician that he or she should advise the patient of the importance of taking the medication. The physician can ask the patient how the discrepancies occurred and work with him or her to find solutions to keep to the times the medication is taken in the future. If necessary, the times at which a medication should be taken can be shifted.

The second computer system (1-2) can remind the patient to perform the mobility test(s).

The second computer system (1-2) can monitor the performance of the mobility test(s) and determine one or more mobility parameters.

The second computer system (1-2) can determine a deviation between the one or more mobility parameters and the one or more thresholds determined by the physician.

The second computer system (1-2) can output the one or more mobility parameters to the patient and/or store the one or more mobility parameters in a data memory and/or transmit the one or more mobility parameters to a separate computer system (e.g., the first computer system). The data memory may be a component of the second computer system (1-2) or a data memory on a cloud server accessible via the network.

In the event of a defined deviation between the one or more mobility parameters and the one or more thresholds, the second computer system (1-2) may transmit a message over the network to the first computer system (1-1).

The second computer system (1-2) can present the specified questions to the patient at the specified times or when the specified events occur and prompt the patient to answer the questions. The second computer system (1-2) may capture the responses, store the responses in a data memory, and/or transmit the responses to a separate computer system. The second computer system (1-2) may also capture and store the times at which the questions were answered.

The second computer system (1-2) may use one or more sensors, which may be part of or connected to the second computer system (1-2) via a wireless connection, to detect the patient's activity and generate an activity profile and/or sleep profile. The second computer system (1-2) may output the activity data and/or activity profile and/or sleep profile to the patient, store it in a data memory, and/or transmit it to a separate computer system.

The second computer system (1-2) can transmit captured, determined, and/or stored data to the first computer system (1-1) via the network at specified times and/or after confirmation by the patient.

If there is only one computer system, the patient may give the computer system to the physician during a visit.

The physician can have the transmitted data (or data stored on the one computer system) output and determine the course of the disease on the basis of the data and check the success of the therapy. The physician can make changes to the therapy, for example, increase or decrease the amount of the medication to be taken and/or increase or decrease the frequency of taking the medication and/or prescribe a different and/or additional medication. The physician may enter new and/or changed specifications for the patient into the first computer system, which are then transmitted over the network to the second computer system. If there is only one computer system, the physician may enter new and/or changed specifications for the patient into the one computer system.

Fig. 2 illustrates a computer system (1) according to some example implementations of the present disclosure in more detail.

Generally, a computer system of exemplary implementations of the present disclosure may be referred to as a computer and may comprise, include, or be embodied in one or more fixed or portable electronic devices. The computer may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs, which may be stored onboard the processing unit (20) or otherwise stored in the memory (50) of the same or another computer.

The processing unit (20) may be a number of processors, a multi-core processor or some other type of processor, depending on the particular implementation. For example, it may be a central processing unit (CPU), a field programmable gate array (FPGA), a graphics processing unit (GPU) and/or a tensor processing unit (TPU). Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program to perform one or more functions, the processing unit (20) of various examples may be capable of performing one or more functions without the aid of a computer program. In either instance, the processing unit (20) may be appropriately programmed to perform functions or operations according to example implementations of the present disclosure.

The memory (50) is generally any piece of computer hardware that is capable of storing information such as, for example, data, computer programs (e. g., computer-readable program code (60)) and/or other suitable information either on a temporary basis and/or a permanent basis. The memory (50) may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium or data memory. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display (screen) may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions (60) may be stored in memory (50), and executed by processing unit (20) that is thereby programmed, to implement functions of the systems, subsystems, tools and their respective elements described herein. As will be appreciated, any suitable program code instructions (60) may be loaded onto a computer or other programmable apparatus from a computer-readable storage medium to produce a particular machine, such that the particular machine becomes a means for implementing the functions specified herein. These program code instructions (60) may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions (60) may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions (60) may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions (60) may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code instructions (60) stored in the memory (50). It will also be understood that one or more functions, and combinations of functions, may be implemented by special purpose hardware-based computer systems and/or processing circuitry which perform the specified functions, or combinations of special purpose hardware and program code instructions.

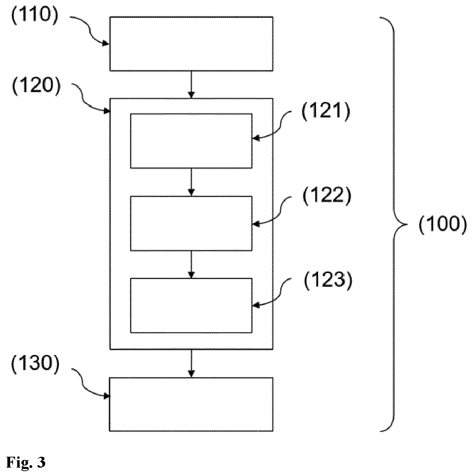

Fig. 3 schematically shows an embodiment of the computer-implemented method of the present disclosure for monitoring the health status of a Parkinson's disease patient.

The method (100) comprises:
(110) receiving specifications for a Parkinson's patient, wherein the specifications were defined by a physician based on an individual therapy for the patient, wherein the specifications contain one or more threshold values,
(120) at repeating points in time:
   (121) outputting a message to the patient, the message comprising a request to perform a mobility test,
   (122) determining and/or receiving one or more mobility parameters as a result of the performance of the mobility test,
   (123) comparing the one or more mobility parameters with the one or more threshold values, thereby determining one or more deviations between the one or more mobility parameters and the one or more threshold values,
(130) making available the mobility parameters and/or the deviations to the physician by outputting and/or storing the mobility parameters and/or the deviations, and/or by transmitting the mobility parameters and/or the deviations to a separate computer system.

## Claims

1. A computer-implemented method, the method comprising:
• receiving specifications for a Parkinson's patient, wherein the specifications were defined by a physician based on an individual therapy for the patient, wherein the specifications contain one or more threshold values,
• at repeating points in time:
∘ outputting a message to the patient, the message comprising a request to perform a mobility test,
∘ determining and/or receiving one or more mobility parameters as a result of the performance of the mobility test,
∘ comparing the one or more mobility parameters with the one or more threshold values, thereby determining one or more deviations between the one or more mobility parameters and the one or more threshold values,
• making available the mobility parameters and/or the deviations to the physician by outputting and/or storing the mobility parameters and/or the deviations, and/or by transmitting the mobility parameters and/or the deviations to a separate computer system.

2. The method of claim 1, further comprising:
• in case of a defined deviation between the one or more mobility parameters and the one or more threshold values: providing a message to the physician about the deviation.

3. The method of claim 1 or 2, wherein the mobility test is atimed up and go test which involves standing up from a seated position in a chair with armrests, walking a defined distance in a direction away from the chair, turning around, returning to the chair, and sitting down again.

4. The method of any one of claims 1 to 3, wherein the one or more thresholds include a maximum duration that the patient should not exceed when performing the mobility test and the one or more mobility parameters include a period of time required for the patient to complete the mobility test.

5. The method of claim 4, wherein the defined deviation is that the period of time is greater than the maximum duration.

6. The method of any one of claims 1 to 5, wherein the specifications further comprise times at which the patient should take a medication.

7. The method of any one of claims 1 to 6, further comprising:
• at repeating points in time:
∘ outputting a message to the patient, where the message reminds the patient to take a medication,
∘ receiving confirmation from the patient that the patient has taken the medication,
∘ storing the confirmation and the time of the confirmation,
• providing the confirmation and the time of the confirmation to the physician.

8. The method of any one of claims 1 to 7, further comprising:
• at repeating points in time:
∘ outputting questions about the quality of the patient's life to the patient,
∘ receiving answers to the questions from the patient,
∘ storing the answers,
∘ providing the answers to the physician.

9. The method of any one of claims 1 to 8, further comprising:
• at repeating points in time:
∘ receiving activity data from one or more sensors, the activity data comprising data on the patient's daily and/or nocturnal activity,
∘ assigning the activity data to an activity profile and/or sleep profile,
∘ storing the activity data and/or the activity profile and/or the sleep profile,
• providing the activity data and/or the activity profile and/or the sleep profile to the physician.

10. A computer system, the computer system comprising: a processor, an input unit, an output unit, and a memory storing an application program configured to perform, when executed by the processor, an operation, the operation comprising:
• receiving specifications for a Parkinson's patient, wherein the specifications were defined by a physician based on an individual therapy for the patient, wherein the specifications contain one or more threshold values,
• at repeating points in time:
∘ outputting a message to the patient, the message comprising a request to perform a mobility test,
∘ determining and/or receiving one or more mobility parameters as a result of the performance of the mobility test,
∘ comparing the one or more mobility parameters with the one or more threshold values, thereby determining one or more deviations between the one or more mobility parameters and the one or more threshold values,
• making available the mobility parameters and/or the deviations to the physician by outputting and/or storing the mobility parameters and/or the deviations, and/or by transmitting the mobility parameters and/or the deviations to a separate computer system.

11. A system comprising a first computer system and a second computer system,
• wherein the first computer system is configured to receive specifications for a Parkinson's patient, wherein the specifications contain one or more threshold values,
• wherein the first computer system is configured to transmit the specifications to the second computer system,
• wherein the second computer system is configured to receive the specifications from the first computer system,
• wherein the second computer system is configured to
∘ output a message to the patient at repeating points in time, the message comprising a request to perform a mobility test,
∘ determine and/or receiving at repeating points in time one or more mobility parameters as a result of the performance of the mobility test,
∘ compare the one or more mobility parameters with the one or more threshold values, thereby determining one or more deviations between the one or more mobility parameters and the one or more threshold values,
∘ transmit the mobility parameters and/or the deviations to the first computer system,
• wherein the first computer system is configured to receive the mobility parameters and/or the deviations from the second computer system,
• wherein the first computer system is configured to output the mobility parameters and/or the deviations to a physician.

12. A non-transitory computer readable medium having stored thereon software instructions that, when executed by a processor of a computer system, cause the computer system to execute the following steps:
• receiving specifications for a Parkinson's patient, wherein the specifications were defined by a physician based on an individual therapy for the patient, wherein the specifications contain one or more threshold values,
• at repeating points in time:
∘ outputting a message to the patient, the message comprising a request to perform a mobility test,
∘ determining and/or receiving one or more mobility parameters as a result of the performance of the mobility test,
∘ comparing the one or more mobility parameters with the one or more threshold values, thereby determining one or more deviations between the one or more mobility parameters and the one or more threshold values,
• making available the mobility parameters and/or the deviations to the physician by outputting and/or storing the mobility parameters and/or the deviations, and/or by transmitting the mobility parameters and/or the deviations to a separate computer system.
